# EUROPÄISCHE PATENTSCHRIFT

(11) **EP 4 432 962 B1**
(45) Veröffentlichungstag und Bekanntmachung des Hinweises auf die Patenterteilung: **17.09.2025**
(21) Anmeldenummer: 22818251.5
(22) Anmeldetag: 14.11.2022
(51) Int. Cl.: A61B 90/98, A61N 5/10, G16H 10/60, A61B 50/00

(54) **KOMPONENTENANORDNUNG FÜR EINE KOMPONENTE AUS DER RÖNTGENTHERAPIE, RÖNTGENTHERAPIESYSTEM UND VERFAHREN ZUM BETREIBEN EINES RÖNTGENTHERAPIESYSTEMS**
COMPONENT ARRANGEMENT FOR AN X-RAY THERAPY COMPONENT, X-RAY THERAPY SYSTEM, AND METHOD FOR OPERATING AN X-RAY THERAPY SYSTEM
AGENCEMENT DE COMPOSANTS POUR COMPOSANT DE RADIOTHÉRAPIE, SYSTÈME DE RADIOTHÉRAPIE ET PROCÉDÉ DE FONCTIONNEMENT D'UN SYSTÈME DE RADIOTHÉRAPIE

(30) Priorität: 15.11.2021 DE 102021212808
(43) Veröffentlichungstag der Anmeldung: 25.09.2024
(73) Patentinhaber: Carl Zeiss Meditec AG, 07745 Jena (DE)
(72) Erfinder: WEIGAND, Frank, 89522 Heidenheim (DE)
(74) Vertreter: Patentanwälte Bressel und Partner mbB
(86) Internationale Anmeldenummer: PCT/EP2022/081833
(87) Internationale Veröffentlichungsnummer: WO 2023/084091

(56) Entgegenhaltungen:
- WO-A1-2015/066814
- DE-A1- 102013 004 168
- DE-B3- 102020 104 628
- US-A1- 2019 290 392

## Beschreibung

Die Erfindung betrifft eine Komponentenanordnung für eine Komponente aus der Röntgentherapie, ein Röntgentherapiesystem und ein Verfahren zum Betreiben eines Röntgentherapiesystems.

Im Bereich der Röntgentherapie ist es für verwendete Komponenten wichtig zu wissen, ob diese bereits verwendet wurden oder nicht, da eine Mehrfachverwendung, z.B. an verschiedenen Systemen und/oder Standorten, zu einem Risiko für den Patienten führen kann. Allgemein besitzen Komponenten aus Kunststoff nur eine begrenzte Toleranz hinsichtlich ionisierender Strahlung, das heißt, diese können zugesicherte Eigenschaften bei Überschreiten der applizierten Dosis verlieren. Ferner müssen im Rahmen einer Behandlungsplanung und Behandlungsvorbereitung Komponenten mit vorgegebenen Eigenschaften bereitgestellt werden. Es ist bekannt, medizinische Komponenten mit Hilfe von Radiofrequenz-Identifikations-(RFID)-Transpondern (RFID-Tags) zu markieren, sodass diese eindeutig identifiziert werden können.

Aus der WO 2009/052048 A1 sind ein System und ein Verfahren zum Bereitstellen einer Strahlentherapie für einen Patienten bekannt. Das System umfasst mindestens eine mit einem RFID-Tag versehene Komponente, z. B. eine Patientenpositionierungsvorrichtung, die verwendet wird, wenn der Patient einer Strahlentherapiebehandlung unterzogen werden soll. Die mindestens eine Komponente wird speziell für die Verwendung beim Patienten ausgewählt. Der RFID-Tag enthält patientenrelevante Informationen. Der RFID-Tag wird von einem Lesegerät abgefragt, um ein Signal zu erzeugen, das von einem zugehörigen Computersystem verwendet wird, um die Position und Anwesenheit des Patienten und der Komponenten in dem Raum, in dem die Behandlung durchgeführt werden soll, zu überprüfen. Das System und das Verfahren ermöglichen es, den Standort jedes Patienten, des ausgewählten Einrichtungspersonals und aller Komponenten für die Behandlung eines bestimmten Patienten zu bestimmen. Außerdem ermöglichen das System und das Verfahren, die Zeit zu bestimmen, die der Patient und/oder das Personal und/oder die Komponente für eine bestimmte Behandlung an einem gegebenen Ort verbringt.

Aus der WO 2014/071337 A1 sind Systeme und Verfahren zum Markieren und Verfolgen von chirurgischen Geräten unter Verwendung von Radiofrequenz-Identifikations-(RFID)-Tags bekannt. Im Allgemeinen ermöglichen die Systeme und Verfahren die Verfolgung chirurgischer Geräte während der gesamten Verteilung und Sterilisation derselben. In einem Beispiel umfasst das System einen Instrumenten-Tray, der konfiguriert ist, um eine Vielzahl von chirurgischen Geräten aufzunehmen, und an dem ein übergeordnetes RFID-Tag angebracht ist, das Informationen enthält und/oder den Zugriff auf Informationen über den Tray und jedes der darin platzierten chirurgischen Geräte ermöglicht. An jedem der chirurgischen Geräte, die in dem Instrumenten-Tray sitzen, kann ein untergeordneter RFID-Tag angebracht sein, der Informationen enthält und/oder den Zugang zu Informationen über das chirurgische Gerät ermöglicht.

Aus der DE 10 2020 104 628 B3 ist ein Applikator zur intraoperativen Radiotherapie bekannt. Der Applikator weist eine Aufnahme zum Aufnehmen einer Bestrahlungssonde und eine Einführöffnung zum Einführen der Bestrahlungssonde in die Aufnahme auf. Die Einführöffnung ist mit einem Siegel verschlossen. Außerdem ist ein erster maschinell auslesbarer Informationsspeicher vorhanden, der zumindest teilweise derart an dem Siegel angeordnet ist, dass ein Entfernen oder Zerstören des Siegels die Auslesbarkeit des Informationsspeichers beseitigt.

Im Rahmen der Röntgentherapie ist eine Handhabung von sterilen Komponenten (beispielsweise von Applikatoren) im Rahmen einer Behandlungsplanung und Behandlungsvorbereitung in nicht sterilen Bereichen nur unbefriedigend gelöst.

Der Erfindung liegt die Aufgabe zu Grunde, eine Handhabung von sterilen Komponenten der Röntgentherapie für eine Behandlungsplanung und Behandlungsvorbereitung zu verbessern.

Die Aufgabe wird erfindungsgemäß durch eine Komponentenanordnung für eine Komponente aus der Röntgentherapie mit den Merkmalen des Patentanspruchs 1, ein Röntgentherapiesystem mit den Merkmalen des Patentanspruchs 6 und ein Verfahren zum Betreiben eines Röntgentherapiesystems mit den Merkmalen des Patentanspruchs 8 gelöst. Vorteilhafte Ausgestaltungen der Erfindung ergeben sich aus den Unteransprüchen.

Es ist einer der Grundgedanken der Erfindung, neben einem an oder in einer sterilen Komponente angeordneten (ersten) RFID-Transponder (welcher auch als RFID-Tag bezeichnet werden kann) einen (zweiten) RFID-Transponder in oder an einer nicht-sterilen Sekundärverpackung anzuordnen. Die sterile Komponente befindet sich in einer Primärverpackung. Die Primärverpackung und die sterile Komponente können dann unabhängig von der Sekundärverpackung bewegt und im Rahmen einer Behandlungsplanung und -vorbereitung gehandhabt werden. Insbesondere ist vorgesehen, dass die sterile Komponente im Auslieferungszustand vom Hersteller in der Primärverpackung und die Primärverpackung in der nicht-sterilen Sekundärverpackung angeordnet ist. Für eine Behandlungsplanung kann der (zweite) RFID-Transponder an der Sekundärverpackung mittels eines (zweiten) RFID-Lesers ausgelesen werden. Dies erfolgt an einer Bedieneinrichtung eines Röntgentherapiesystems, die sich insbesondere in einem nicht-sterilen Bereich eines Operationssaals befindet. Probleme mit der Sterilität ergeben sich nicht, da die Komponente weiterhin steril in der Primärverpackung verpackt bleibt. Die Behandlungsplanung wird an der Bedieneinrichtung beispielsweise von einem Medizinphysiker oder sonstigem Fachpersonal durchgeführt. Die Primärverpackung kann anschließend aus der Sekundärverpackung entnommen werden und die sterile Komponente wird in einen sterilen Bereich des Operationssaals verbracht. Dort wird der an oder in der Komponente angeordnete (erste) RFID-Transponder mittels eines an einer Röntgentherapieeinrichtung des Röntgentherapiesystems angeordneten (ersten) RFID-Lesers ausgelesen, um beispielsweise eine Freigabe der Röntgenquelle bzw. Röntgenstrahlung zu veranlassen und/oder einen Sperrzustand der Röntgenquelle bzw. der Röntgenstrahlung aufzuheben.

Insbesondere wird eine Komponentenanordnung für eine Komponente aus der Röntgentherapie geschaffen, umfassend eine Komponente für die Röntgentherapie mit einem ersten RFID-Transponder, eine Primärverpackung, in der die Komponente steril verpackt ist, und eine Sekundärverpackung mit einem darin und/oder daran angeordneten zweiten RFID-Transponder, in der die in der Primärverpackung verpackte Komponente angeordnet ist.

Ferner wird insbesondere ein Röntgentherapiesystem geschaffen, umfassend eine Röntgentherapieeinrichtung mit einer Halteeinrichtung, die zum Halten und/oder Positionieren mindestens einer Komponente in und/oder an einem Patienten eingerichtet ist, eine Bedieneinrichtung, die zum Steuern der Röntgentherapieeinrichtung eingerichtet ist, wobei die Röntgentherapieeinrichtung mindestens einen ersten RFID-Leser umfasst, der dazu eingerichtet ist, Daten eines an oder in der mindestens einen Komponente angeordneten ersten RFID-Transponders auszulesen und/oder diesen mit Daten zu beschreiben, und wobei die Bedieneinrichtung mindestens einen zweiten RFID-Leser umfasst, der dazu eingerichtet ist, Daten eines in und/oder an einer Sekundärverpackung angeordneten zweiten RFID-Transponders auszulesen und/oder diesen mit Daten zu beschreiben.

Weiter wird insbesondere ein Verfahren zum Betreiben eines Röntgentherapiesystems zur Verfügung gestellt, wobei zum Einrichten des Röntgentherapiesystems für jede von mindestens einer Komponente: Daten eines in und/oder an einer Sekundärverpackung der Komponente angeordneten zweiten RFID-Transponders mittels eines zweiten RFID-Lesers einer Bedieneinrichtung des Röntgentherapiesystems ausgelesen werden und/oder der zweite RFID-Transponder mit Daten beschrieben wird, die in einer Primärverpackung steril verpackte Komponente aus der Primärverpackung entnommen wird, und Daten eines ersten RFID-Transponders der Komponente mittels eines ersten RFID-Lesers einer Röntgentherapieeinrichtung des Röntgentherapiesystems ausgelesen werden und/oder der erste RFID-Transponder mit Daten beschrieben wird.

Die Komponentenanordnung, das Röntgentherapiesystem und das Verfahren ermöglichen eine verbesserte Handhabung von Komponenten aus der Röntgentherapie im Rahmen einer Behandlungsplanung und -vorbereitung. Durch das Verwenden von zwei RFID-Transpondern an oder in einer Sekundärverpackung und an oder in der Komponente wird insbesondere auch das Problem gelöst, dass der an oder in der Komponente angeordnete RFID-Transponder in der Regel nicht im verpackten Zustand der Komponente ausgelesen werden kann, da ein Abstand zwischen dem RFID-Transponder und einem RFID-Leser unter den gegebenen Umständen zu groß ist. Ferner kann die Sekundärverpackung von der Primärverpackung und der sterilen Komponente örtlich getrennt werden, sodass eine Behandlungsplanung und/oder eine Behandlungsvorbereitung ohne die sterile Komponente selbst an der (nicht-sterilen) Bedieneinrichtung des Röntgentherapiesystems durchgeführt werden kann.

Die Komponente ist insbesondere eine Komponente für die Röntgentherapie. Beispielsweise kann die Komponente ein Applikator sein, der bei einer Behandlung an einer Röntgenquelle angeordnet wird oder angeordnet ist, und der zum gezielten Verabreichen und Abschirmen von Röntgenstrahlung dient.

Die Bedieneinrichtung ist insbesondere separat von der Röntgentherapieeinrichtung ausgebildet. Die Bedieneinrichtung und die Röntgentherapieeinrichtung sind jedoch signaltechnisch miteinander verbunden. Die Bedieneinrichtung umfasst insbesondere eine Steuereinrichtung zum Steuern einer Behandlung des Patienten mit der Röntgentherapieeinrichtung. Die von dem zweiten RFID-Transponder ausgelesenen Daten werden insbesondere in der Bedieneinrichtung, insbesondere mittels der Steuereinrichtung, für eine Behandlungsplanung ausgewertet. Insbesondere können hierbei Konfigurationsparameter für die Röntgentherapieeinrichtung ausgehend von den ausgelesenen Daten erzeugt werden.

Die aus den RFID-Transpondern ausgelesenen und/oder auf diese geschriebenen Daten können beispielsweise die folgenden Daten umfassen: einen Benutzungszustand (z.B. benutzt/unbenutzt/steril), eine Anzahl von Benutzungen, ein Datum und eine Uhrzeit einer erfolgten Operation, eine applizierte Dosis in Gy/min, ein Name eines Krankenhauses, ein Name des operierenden Arztes, ein Name des Medizinphysikers usw.

Teile der Bedieneinrichtung und/oder der Röntgentherapieeinrichtung, insbesondere eine oder mehrere Steuereinrichtungen, können einzeln oder zusammengefasst als eine Kombination von Hardware und Software ausgebildet sein, beispielsweise als Programmcode, der auf einem Mikrocontroller oder Mikroprozessor ausgeführt wird. Es kann jedoch auch vorgesehen sein, dass Teile einzeln oder zusammengefasst als anwendungsspezifische integrierte Schaltung (ASIC) und/oder feldprogrammierbares Gatterfeld (FPGA) ausgebildet sind.

Die Röntgentherapieeinrichtung umfasst eine Halteeinrichtung, welche beispielsweise als Stativ ausgebildet ist oder ein Stativ umfasst. Grundsätzlich kann die Halteeinrichtung jedoch auch anders ausgebildet sein und beispielsweise als Haltearm ausgebildet sein oder einen Haltearm umfassen. Ein solcher Haltearm kann beispielsweise an einer Patientenliege angeordnet sein. Ferner umfasst die Röntgentherapieeinrichtung insbesondere eine Röntgenquelle, die an der Halteeinrichtung, insbesondere an einem (distalen) Ende eines Stativs oder Haltearms, angeordnet ist. An der Röntgenquelle kann als Komponente ein (Röntgen-)Applikator angeordnet sein bzw. angeordnet werden. Vor Durchführen einer Behandlung mittels Röntgenstrahlung der Röntgenquelle wird insbesondere festgestellt, ob der erste RFID-Transponder einer für die Behandlung vorgesehenen Komponente, beispielsweise eines an der Röntgenquelle angeordneten Applikators, von dem ersten RFID-Leser erfasst wird. Grundsätzlich kann die Anwesenheit weiterer vorgegebener Komponenten überwacht und/oder überprüft werden, beispielsweise einer Halteplatte etc. Nur wenn die Anwesenheit der Komponente erfasst wurde, wird die Behandlung freigegeben, beispielsweise indem die Röntgenquelle mit Hilfe eines Freigabesignals freigegeben wird oder indem ein Sperrsignal aufgehoben wird. Der erste RFID-Transponder und der erste RFID-Leser bilden zusammen insbesondere ein Sperrsystem (engl. Interlock) aus, mit dem ein Betrieb der Röntgenquelle überwacht und gesichert wird.

Eine Primärverpackung ist insbesondere eine verschweißte Hülle (Peel Pouch) oder ein mit einer Sterilbarriere (z.B. Tyvek^{®}-Siegel) versiegelter Blister, in der bzw. in dem die sterile Komponente angeordnet ist.

Eine Sekundärverpackung ist insbesondere eine nicht-sterile Kartonage aus Karton und/oder Pappe. Die Sekundärverpackung ist insbesondere als Schachtel oder Faltschachtel ausgebildet. Der zweite RFID-Transponder ist beispielsweise in Form eines Etiketts auf einer Innenseite oder einer Außenseite der Sekundärverpackung angeordnet, beispielsweise aufgeklebt. Eine in der Sekundärverpackung angeordnete Primärverpackung beinhaltet insbesondere nur eine Komponente. Anders ausgedrückt ist jeder Komponente insbesondere eine Primärverpackung und eine Sekundärverpackung zugeordnet.

In einer Ausführungsform ist vorgesehen, dass der erste RFID-Transponder und/oder der zweite RFID-Transponder eindeutige Komponentenidentifikationsdaten bereitstellen. Insbesondere kann dies eine eindeutige Seriennummer sein, insbesondere ein UID (Abkürzung für engl. Unique Identifier). Hierdurch lässt sich eine Komponente eindeutig identifizieren. Die eindeutigen Komponentenidentifikationsdaten sind insbesondere in einem Speicher des jeweiligen RFID-Transponders hinterlegt und können mittels eines RFID-Lesers aus diesem abgerufen werden. Über die eindeutigen Komponentenidentifikationsdaten können beispielsweise weitere Informationen zu der mindestens einen Komponente aus einer Datenbank abgerufen werden.

In einer Ausführungsform ist vorgesehen, dass der erste RFID-Transponder biokompatibel ist. Hierdurch kann der erste RFID-Transponder mit Gewebe des Patienten in Kontakt kommen, ohne dass es zu Beeinträchtigungen kommt. Hierzu ist der erste RFID-Transponder beispielsweise eingebettet in Glas oder in einem geeigneten Kunststoff.

In einer Ausführungsform ist vorgesehen, dass der erste RFID-Transponder mittels eines Sterilisationsverfahrens sterilisierbar ist. Hierdurch kann die Komponente mit dem ersten RFID-Transponder nach einer Benutzung wieder sterilisiert werden und hierdurch erneut benutzt werden. Das Sterilisationsverfahren kann beispielsweise eine Dampfsterilisierung und/oder eine Gamma-Bestrahlung umfassen.

In der nachfolgenden Tabelle sind beispielhaft RFID-Transponder aufgelistet, die verwendet werden können:

| **Hersteller** | **Modell** | **Frequenz** | **Speicher** | **Anwendungsort** | **Verkapselung** | **Sterilisierbarkeit** |
|---|---|---|---|---|---|---|
| HIDGlobal | 6B0201 | 13,56 MHz | 1664 Bit | Glas, Metall, Plastik, Holz | Glasröhre | Dampf |
| HIDGlobal | In Tag 200 | 13,56 MHz | 16 kBit | Glas, Plastik, Holz | Kunststoff | Gamma |
| HIDGlobal | Piccolino Tag 634190 | 13,56 MHz | 16 kBit | k.A. | Epoxydharz | Dampf |
| Neosid | 704032 | 13,56 MHz | 896 Bit | nichtmetallische Objekte | Kunststoff | Dampf |
| Neosid | 704033 | 13,56 MHz | 896 Bit | metallische Objekte | Kunststoff | Dampf |
| Xerafy | Dot-iN XS | 866-868 MHz | 512 Bit | Glas | Keramik | Dampf |
| Xerafy | Pico-iN Plus | 866-868 MHz | 512 Bit | k.A. | Keramik | Dampf |
| Xerafy | Dash-iN XS | 866-868 MHz | 512 Bit | Glas, Metall, Plastik, Holz | Keramik | Dampf |

In einer Ausführungsform ist vorgesehen, dass die Komponente ein Applikator für die Röntgentherapie ist. Der Applikator wird zur Nutzung an bzw. auf einer Röntgenquelle des Röntgentherapiesystems angeordnet. Der Applikator dient insbesondere dazu, zu bestrahlendes Gewebe in einem vorgegebenen Abstand zu einem Ausgangspunkt (d.h. zum Target) der Röntgenstrahlung anzuordnen sowie nicht zu bestrahlendes Gewebe zu schützen.

In einer Ausführungsform ist vorgesehen, dass der mindestens eine erste RFID-Leser an der Halteeinrichtung, beispielsweise an einem Stativ, in einem Verbindungsbereich angeordnet ist, in dem die mindestens eine Komponente zur Nutzung an der Halteeinrichtung angeordnet ist oder angeordnet werden kann. Hierdurch kann insbesondere auch bei einer begrenzten Sendeleistung des ersten RFID-Lesers eine Kommunikationsverbindung zu dem ersten RFID-Transponder ausgebildet werden. Durch das Anordnen des ersten RFID-Lesers und das Begrenzen der Sendeleistung des ersten RFID-Lesers kann insbesondere sichergestellt werden, dass die Komponente nur erfasst wird, wenn diese in dem Verbindungsbereich angeordnet ist. Dies ermöglicht insbesondere das Bereitstellen eines Sperrsystems (engl. Interlock), bei dem die Röntgenquelle und eine Röntgenstrahlung nur freigegeben wird, wenn eine vorgegebene Komponente (oder mehrere vorgegebene Komponenten) in dem Verbindungsbereich angeordnet sind. Beispielsweise kann vorgesehen sein, die Röntgenquelle nur für einen Betrieb freizugeben, wenn ein Applikator an der Röntgenquelle angeordnet ist. Der Verbindungsbereich und eine Sendeleistung des ersten RFID-Lesers werden dann derart gewählt, dass nur bei Anordnung des Applikators an der Röntgenquelle im Bereich des Verbindungsbereichs ein Abstand zwischen dem ersten RFID-Transponder und dem ersten RFID-Leser klein genug ist, dass eine Kommunikationsverbindung zwischen diesen ausgebildet werden kann und die Daten aus dem ersten RFID-Transponder ausgelesen werden können.

In einer Ausführungsform ist vorgesehen, dass eine Freigabe der Röntgentherapie erfolgt, wenn die Daten des ersten RFID-Transponders der mindestens einen Komponente mindestens eine vorgegebene Information umfassen. Hierdurch kann sichergestellt werden, dass die mindestens eine Komponente vorgegebene Eigenschaften aufweist (z.B. Benutzungszustand, Anzahl der Benutzungen, vorgegebener Typ). Eine Fehlbedienung und/oder eine Fehlnutzung kann hierdurch verhindert werden. Beispielsweise kann vorgesehen sein, dass eine Nutzung der Röntgentherapieeinrichtung verhindert wird, wenn nach dem Auslesen des ersten RFID-Transponders festgestellt wird, dass die mindestens eine Information nicht vorhanden ist. Die mindestens eine Information kann beispielsweise eine eindeutige Seriennummer und/oder eine Typennummer umfassen, insbesondere ein UID (engl. Unique Identifier). Vor dem Benutzen einer Komponente kann beispielsweise überprüft werden, ob die Komponente bereits benutzt wurde oder nicht (Benutzungszustand), indem die entsprechende Information ausgelesen und überprüft wird. Entsprechend kann nach jeder Benutzung (vgl. nachfolgend beschriebene Ausführungsform) eine Information in dem Speicher des ersten RFID-Transponders hinterlegt sein, dass die Komponente bereits benutzt ist (bzw. unbenutzt ist). Ferner kann beispielsweise überprüft werden, ob eine Maximalnutzungsanzahl bereits erreicht ist oder nicht (Anzahl der Benutzungen). Hierzu kann eine in einem Speicher des ersten RFID-Transponders hinterlegte Anzahl der Benutzungen, nachdem diese ausgelesen wurde, beispielsweise mit einem Maximalwert verglichen werden. Unter Berücksichtigung von einem Vergleichsergebnis wird dann ein Freigabesignal für die Röntgenquelle und/oder die Behandlung erzeugt oder nicht.

In einer Ausführungsform ist vorgesehen, dass nach einem Benutzen der mindestens einen Komponente eine Information in dem ersten RFID-Transponder hinterlegt wird, die die mindestens eine Komponente als benutzt markiert und/oder die eine Anzahl von Benutzungen der mindestens einen Komponente umfasst. Hierdurch kann die mindestens eine Komponente unmittelbar als benutzt gekennzeichnet werden. Vor dem Benutzen einer Komponente kann dann überprüft werden, ob die Komponente bereits benutzt wurde oder nicht, indem die entsprechende Information ausgelesen und überprüft wird. Ferner kann beispielsweise überprüft werden, ob eine Maximalnutzungsanzahl bereits erreicht ist oder nicht. Hierzu kann die hinterlegte Anzahl der Benutzungen, nachdem diese ausgelesen wurde, beispielsweise mit einem Maximalwert verglichen werden. Unter Berücksichtigung von einem Vergleichsergebnis wird dann ein Freigabesignal für die Röntgenquelle und/oder die Behandlung erzeugt oder nicht.

In einer Ausführungsform ist vorgesehen, dass der erste RFID-Transponder nach dem Benutzen und/oder nach Erreichen einer vorgegebenen Anzahl von Benutzungen unbrauchbar gemacht wird. Hierdurch kann der erste RFID-Transponder irreversibel markiert werden. Eine Anzahl von Benutzungen der Komponente kann hierdurch insgesamt begrenzt werden. Dies ist beispielsweise erforderlich, wenn ein Material der Komponente, beispielsweise ein Kunststoff, eine maximal zulässige Gesamtdosis von ionisierender Strahlung erreicht hat. Eine irreversible Zerstörung kann beispielsweise durch Erzeugen eines Sendepulses mit einer vorgegebenen Leistung oberhalb eines geeigneten Schwellwertes erfolgen, der aufgrund einer eingekoppelten Gesamtenergie zu einer thermischen Zerstörung (Durchtrennung und/oder Kurzschluss) von Leiterbahnen des ersten RFID-Transponders führt. Anschließend funktioniert der erste RFID-Transponder nicht mehr und kann nicht mehr ausgelesen werden.

In einer Ausführungsform ist vorgesehen, dass die mindestens eine Komponente nach einer Benutzung sterilisiert wird. Hierdurch kann die mindestens eine Komponente wiederverwendet werden. Der erste RFID-Transponder ist in diesem Fall sterilisierbar ausgebildet und durchläuft zusammen mit der Komponente die Sterilisation. Die Komponente und der erste RFID-Transponder können beispielsweise in einem Sterilisationstray (bzw. Instrumententray) oder einem Peel Pouch angeordnet werden und anschließend ein Sterilisationsverfahren durchlaufen (z.B. Dampfsterilisation oder Gammastrahlen-Sterilisation). Vor dem Anordnen in dem Sterilisationstray oder dem Peel Pouch wird die Komponente durch entsprechendes Beschreiben des ersten RFID-Transponders auf den Zustand "unbenutzt" zurückgesetzt, beispielsweise indem ein hierfür vorgesehenes Bit geändert wird. Die Sekundärverpackung verbleibt außerhalb des Trays oder des Peel Pouches, sodass der zweite RFID-Transponder weiterhin zum Vorbereiten und/oder Planen der Behandlung an der Bedieneinrichtung ausgelesen und/oder beschrieben werden kann. Es kann vorgesehen sein, dass die Komponente nach einer Benutzung und einer anschließenden Reinigung im nicht-sterilen Zustand in der Sekundärverpackung gelagert wird und erst bei erneutem Bedarf sterilisiert wird. Das Vorgehen ist hierbei aber grundsätzlich wie voranstehend beschrieben. Insbesondere erfolgt das Markieren als "unbenutzt" erst kurz vor Anordnen der Komponente in dem Tray oder dem Peel Pouch.

Nachfolgend wird die Erfindung anhand bevorzugter Ausführungsbeispiele unter Bezugnahme auf die Figuren näher erläutert. Hierbei zeigen:
- Fig. 1: eine schematische Darstellung einer Ausführungsform der Komponentenanordnung für eine Komponente aus der Röntgentherapie;
- Fig. 2: eine schematische Darstellung einer Ausführungsform des Röntgentherapiesystems;
- Fig. 3: ein schematisches Ablaufdiagramm einer Ausführungsform des Verfahrens zum Betreiben eines Röntgentherapiesystems.

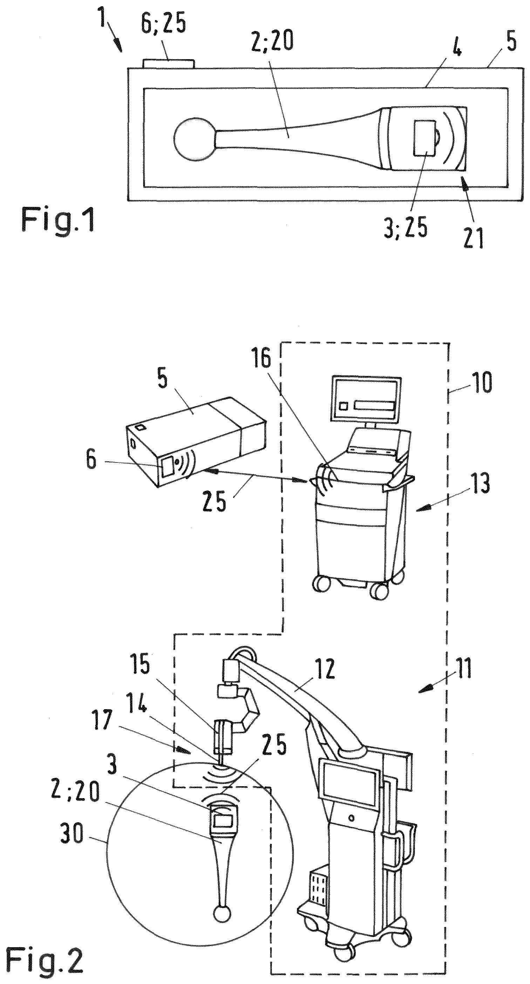

In Fig. 1 ist eine schematische Darstellung einer Ausführungsform der Komponentenanordnung 1 für eine Komponente 2 aus der Röntgentherapie gezeigt.

Die Komponentenanordnung 1 umfasst eine Komponente 2 für die Röntgentherapie mit einem ersten RFID-Transponder 3. Der erste RFID-Transponder 3 ist in oder an der Komponente 2 angeordnet. Die Komponente 2 ist insbesondere als Applikator 20 ausgebildet, der in einem Verbindungsbereich 21 mit einer Röntgenquelle verbunden werden kann (vgl. Fig. 2). Der erste RFID-Transponder 3 ist insbesondere in dem Verbindungsbereich 21 oder in der Nähe des Verbindungsbereichs 21 angeordnet, sodass der erste RFID-Transponder 3 von einem an der Röntgenquelle angeordneten RFID-Leser ausgelesen und/oder beschrieben werden kann.

Ferner umfasst die Komponentenanordnung 1 eine Primärverpackung 4, in der die Komponente 2 steril verpackt ist. Die Primärverpackung 4 kann beispielsweise ein Peel Pouch oder ein mit einer Sterilbarriere (z.B. Tyvek^{®}-Folie) versiegelter Blister sein.

Weiter umfasst die Komponentenanordnung 1 eine Sekundärverpackung 5 mit einem darin und/oder daran angeordneten zweiten RFID-Transponder 6. In der Sekundärverpackung 5 sind die Primärverpackung 4 und die darin steril verpackte Komponente 2 angeordnet. Die Sekundärverpackung 5 ist beispielsweise als Schachtel oder Faltschachtel aus Karton oder Pappe ausgebildet. Die Sekundärverpackung 5 ist nicht-steril und dient der Handhabung, dem Transportschutz und der Lagerung der sterilen Komponente 2 in der Primärverpackung 4.

Es kann vorgesehen sein, dass der erste RFID-Transponder 3 und/oder der zweite RFID-Transponder 6 eindeutige Komponentenidentifikationsdaten 25 bereitstellen. Die eindeutigen Komponentenidentifikationsdaten 25 umfassen insbesondere eine eindeutige Seriennummer (z.B. einen Unique Identifier, UID). Die eindeutigen Komponentenidentifikationsdaten 25 sind hierbei in einem Speicher der RFID-Transponders 3, 6 hinterlegt und können aus diesem bei Bedarf abgerufen und bereitgestellt werden.

Es kann vorgesehen sein, dass der erste RFID-Transponder 3 biokompatibel ist. Dies erlaubt einen Kontakt des ersten RFID-Transponders 3 mit biologischem Gewebe. Hierzu kann der RFID-Transponder 3 beispielsweise in (Bio-)Glas oder einem geeigneten Kunststoff gekapselt sein.

Es kann vorgesehen sein, dass der erste RFID-Transponder 3 mittels eines Sterilisationsverfahrens sterilisierbar ist. Hierzu geeignete RFID-Transponder 3 wurden bereits voranstehend aufgelistet.

In Fig. 2 ist eine schematische Darstellung einer Ausführungsform des Röntgentherapiesystems 10 gezeigt. Das Röntgentherapiesystem 10 umfasst eine Röntgentherapieeinrichtung 11 mit einer Halteeinrichtung 12, die zum Halten und/oder Positionieren mindestens einer Komponente 2 in und/oder an einem Patienten eingerichtet ist. Die Halteeinrichtung 12 ist beispielsweise als Stativ ausgebildet oder umfasst ein Stativ. Ferner umfasst das Röntgentherapiesystem 10 eine Bedieneinrichtung 13, die zum Steuern der Röntgentherapieeinrichtung 11 eingerichtet ist.

Zumindest ein Teilbereich der Halteeinrichtung 12, insbesondere der Teilbereich, an dem die mindestens eine Komponente 2 angeordnet ist oder angeordnet werden kann, befindet sich in einem sterilen Bereich 30 des Operationssaals. Das restliche Röntgentherapiesystem 10, insbesondere die Bedieneinrichtung 13, befindet sich außerhalb des sterilen Bereichs 30 in einem nicht-sterilen Bereich und kann entsprechend einfacher gehandhabt werden.

Die Röntgentherapieeinrichtung 11 umfasst einen ersten RFID-Leser 14, der dazu eingerichtet ist, Daten eines an oder in der mindestens einen Komponente 2 angeordneten ersten RFID-Transponders 3 auszulesen und/oder diesen mit Daten zu beschreiben. Insbesondere ist vorgesehen, dass der erste RFID-Leser 14 an der Halteeinrichtung 12 in einem Verbindungsbereich 17 angeordnet ist, in dem die mindestens eine Komponente 2 zur Nutzung an der Halteeinrichtung 12 angeordnet ist oder angeordnet werden kann. Der RFID-Transponder 3 ist insbesondere in einem Verbindungsbereich 17 angeordnet, in dem die Komponente 2, insbesondere ein Applikator 20, an einer an der Halteeinrichtung 12 angeordneten Röntgenquelle 15 angeordnet ist bzw. angeordnet werden kann.

Die Bedieneinrichtung 13 umfasst einen zweiten RFID-Leser 16, der dazu eingerichtet ist, Daten eines in und/oder an einer Sekundärverpackung 5 angeordneten zweiten RFID-Transponders 6 auszulesen und/oder diesen mit Daten zu beschreiben.

Sowohl die Röntgentherapieeinrichtung 11 als auch die Bedieneinrichtung 13 umfassen jeweils Steuereinrichtungen (nicht gezeigt) mit jeweils einer Recheneinrichtung und einem Speicher. Die Recheneinrichtungen umfassen beispielsweise jeweils einen Mikroprozessor und sind dazu eingerichtet, in dem Speicher hinterlegten Programmcode auszuführen. Die Steuereinrichtungen sind insbesondere dazu eingerichtet, eine Kommunikation zwischen den jeweiligen RFID-Lesern 14, 16 und den RFID-Transpondern 3, 6 zu steuern und Daten, insbesondere eindeutige Komponentenidentifikationsdaten 25, von den jeweiligen RFID-Transpondern 3, 6 auszulesen und auszuwerten und/oder diese mit Daten zu beschreiben. Insbesondere stellt die Steuereinrichtung der Röntgentherapieeinrichtung 11 hierdurch eine Funktionalität einer Sperreinrichtung (engl. Interlock) zur Verfügung, sodass die Röntgentherapieeinrichtung 11 nur betrieben werden kann bzw. eine Behandlung nur freigegeben wird, wenn die mindestens eine vorgegebene Komponente 2 korrekt angeordnet ist und unbenutzt (steril) ist. Insbesondere kann hierdurch sichergestellt werden, dass ein (unbenutzter/steriler) Applikator 20 an der Röntgenquelle 15 angeordnet ist, bevor die Röntgenquelle 15 aktiviert wird. Ferner kann vorgesehen sein, auch eine Funktionalität von anderen Komponenten der Röntgentherapieeinrichtung 11, wie beispielsweise der Halteeinrichtung 12 zum Halten der Röntgenquelle 15 und des Applikators 20, in Abhängigkeit von einem Vorhandensein des Applikators 20 an der Röntgenquelle 15 zu sperren oder freizugeben. Hierdurch kann das Anordnen eines benutzten/nicht-sterilen Applikators 20 am oder im Patienten verhindert werden.

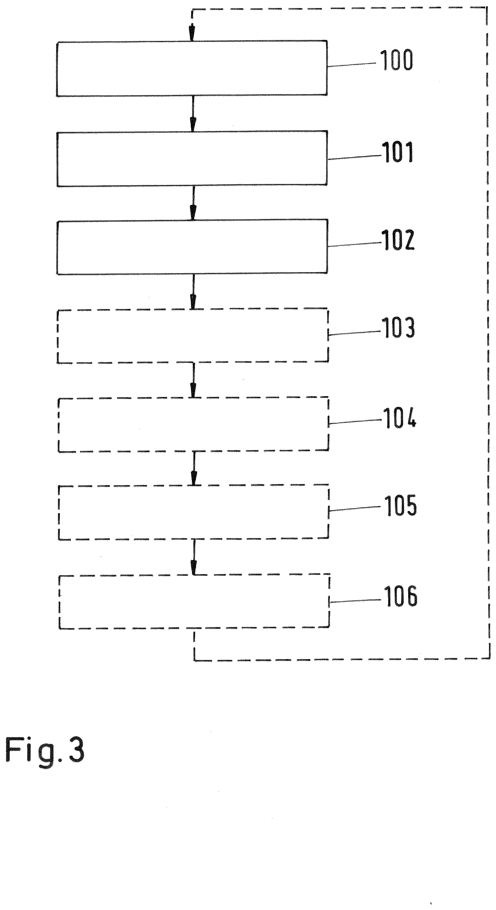

In Fig. 3 ist ein schematisches Ablaufdiagramm einer Ausführungsform des Verfahrens zum Betreiben eines Röntgentherapiesystems 1 gezeigt. Nachfolgend wird ergänzend zur Fig. 3 auch Bezug genommen auf die Fig. 2.

Zum Einrichten des Röntgentherapiesystems 10 wird für jede von mindestens einer Komponente 2 die nachfolgend beschriebenen Maßnahmen ausgeführt. Die Maßnahmen werden hierbei jedoch nur für eine Komponente 2 beschrieben. Grundsätzlich kann das Verfahren jedoch auch für weitere Komponenten 2 in gleicher Weise ausgeführt werden. Die Komponente 2 ist insbesondere ein Applikator 20, der während der Röntgentherapie an der Röntgenquelle 15 der Röntgentherapieeinrichtung 11 angeordnet ist.

In einer Maßnahme 100 werden Daten eines in und/oder an einer Sekundärverpackung 5 der Komponente 2 angeordneten zweiten RFID-Transponders 6 mittels des zweiten RFID-Lesers 16 der Bedieneinrichtung 13 des Röntgentherapiesystems 10 ausgelesen und/oder der zweite RFID-Transponder 6 wird mit Daten beschrieben.

Insbesondere ist hierbei vorgesehen, dass eindeutige Komponentenidentifikationsdaten 25 (z.B. ein Unique Identifier, UID) aus dem zweiten RFID-Transponder ausgelesen wird. Mit Hilfe der ausgelesenen Daten kann eine Behandlung an der Bedieneinrichtung 13 vorbereitet werden. Beispielsweise kann ein Typ und Eigenschaften eines Applikators 20 für einen Behandlungsplan in dem Röntgentherapiesystem 10 hinterlegt werden. Grundsätzlich können auch mehrere gleichartige Komponenten 2 unterschiedlichen Typs (z.B. Applikatoren unterschiedlicher Form) hinterlegt werden, aus denen kurz vor der konkreten Behandlung die verwendete Komponente 2 ausgewählt wird (z.B. wird eine Form des Applikators 20 in Abhängigkeit von einem Operationsverlauf ausgewählt).

In einer Maßnahme 101 wird die in einer Primärverpackung steril verpackte Komponente 2, insbesondere der Applikator 20, aus der Primärverpackung entnommen. Ist die Primärverpackung in der Sekundärverpackung 5 angeordnet, so wird die Primärverpackung zusammen mit der darin angeordneten Komponente 2 aus der Sekundärverpackung 5 entnommen. Das Entnehmen der steril verpackten Komponente 2 aus der Primärverpackung erfolgt hierbei derart, dass eine Sterilität der Komponente 2 hierbei nicht verloren geht.

In einer Maßnahme 102 werden Daten des ersten RFID-Transponders 3 der Komponente 2, insbesondere des Applikators 20, mittels des ersten RFID-Lesers 14 der Röntgentherapieeinrichtung 11 des Röntgentherapiesystems 10 ausgelesen und/oder der erste RFID-Transponder 3 wird mit Daten beschrieben. Insbesondere ist hierbei vorgesehen, dass eindeutige Komponentenidentifikationsdaten 25 (z.B. ein Unique Identifier, UID) aus dem ersten RFID-Transponder 3 ausgelesen wird. Ferner kann vorgesehen sein, dass hierbei Daten ausgelesen werden, die eine Information darüber umfassen, ob die Komponente 2 bereits benutzt ist oder nicht.

Es kann vorgesehen sein, dass in einer Maßnahme 103 eine Freigabe der Röntgentherapie erfolgt, wenn die Daten des ersten RFID-Transponders 3 der Komponente 2, insbesondere des Applikators 20, mindestens eine vorgegebene Information umfassen. Insbesondere ist hierbei vorgesehen, dass eindeutige Komponentenidentifikationsdaten 25 (z.B. ein Unique Identifier, UID) überprüft werden. Alternativ oder zusätzlich kann ein Sperrsignal erzeugt werden, wenn die Daten die mindestens eine vorgegebene Information nicht umfassen. Die mindestens eine vorgegebene Information kann beispielsweise einen Benutzungszustand umfassen, der vorgibt, dass die Komponente 2 unbenutzt sein muss. Beispielsweise kann ein entsprechend hierfür reserviertes Bit überprüft werden. Enthält das Bit die vorgegebene Information (unbenutzt), wird die Behandlung freigegeben, anderenfalls nicht.

Es kann in einer Maßnahme 104 vorgesehen sein, dass nach einem Benutzen der Komponente 2, insbesondere des Applikators 20, eine Information in dem ersten RFID-Transponder 3 hinterlegt wird, die die Komponente 2 als benutzt markiert und/oder die eine Anzahl von Benutzungen der Komponente 2 umfasst. Hierzu werden entsprechende Daten in einen Speicher des ersten RFID-Transponders 3 geschrieben.

Es kann in einer Maßnahme 105 vorgesehen sein, dass der erste RFID-Transponder 3 nach dem Benutzen und/oder nach Erreichen einer vorgegebenen Anzahl von Benutzungen unbrauchbar gemacht wird. Dies erfolgt beispielsweise durch Einkoppeln einer großen Sendeleistung (oberhalb eines geeigneten Schwellwertes), die Leiterbahnen des ersten RFID-Transponders 3 durch thermischen Energieeintrag irreversibel zerstört und/oder kurzschließt.

Es kann in einer Maßnahme 106 vorgesehen sein, dass die Komponente 2, insbesondere der Applikator 20, nach einer Benutzung sterilisiert wird. Hierzu wird die Komponente 2 beispielsweise in einem Sterilisationstray oder einem Peel Pouch angeordnet. Der Sterilisationstray oder das Peel Pouch bilden hierbei nach der Sterilisation insbesondere eine Primärverpackung aus. Hierbei ist insbesondere vorgesehen, dass der erste RFID-Transponder 3 unmittelbar vor dem Sterilisieren mit Daten beschrieben wird, die eine Information umfassen, die die Komponente 2 als unbenutzt und/oder als steril markiert. Beispielsweise kann ein vorher (d.h. nach erfolgter Benutzung) geändertes Bit wieder zurück geändert werden. Anschließend können die voranstehenden Maßnahmen mit der sterilisierten Komponente 2 erneut durchgeführt werden.

Das in dieser Offenbarung beschriebene Verfahren verbessert eine Handhabung von Komponenten der Strahlentherapie. Insbesondere kann ein Vorbereiten und Arbeiten sowohl im nicht-sterilen als auch im sterilen Bereich vereinfacht werden. Eine Behandlungsplanung und eine Behandlungsvorbereitung können hierdurch verbessert, insbesondere vereinfacht, werden. Insbesondere ist ein Arbeitsfluss verbessert, da ein schnelles und unkompliziertes Erfassen und/oder Schreiben von Daten in Bezug auf die mindestens eine Komponente sowohl im nicht-sterilen als auch im sterilen Bereich eines Operationssaals unabhängig voneinander möglich ist.

### Bezugszeichenliste

- 1: Komponentenanordnung
- 2: Komponente
- 3: erster RFID-Transponder
- 4: Primärverpackung
- 5: Sekundärverpackung
- 6: zweiter RFID-Transponder
- 10: Röntgentherapiesystem
- 11: Röntgentherapieeinrichtung
- 12: Halteeinrichtung
- 13: Bedieneinrichtung
- 14: erster RFID-Leser
- 15: Röntgenquelle
- 16: zweiter RFID-Leser
- 17: Verbindungsbereich
- 20: Applikator
- 21: Verbindungsbereich
- 25: Komponentenidentifikationsdaten
- 30: steriler Bereich
- 100-106: Maßnahmen des Verfahrens

## Patentansprüche

1. Komponentenanordnung (1) für eine Komponente (2) aus der Röntgentherapie, umfassend:
eine Komponente (2) für die Röntgentherapie mit einem ersten RFID-Transponder (3),
eine Primärverpackung (4), in der die Komponente (2) steril verpackt ist,
**gekennzeichnet durch**
eine Sekundärverpackung (5) mit einem darin und/oder daran angeordneten zweiten RFID-Transponder (6), in der die in der Primärverpackung (4) verpackte Komponente (2) angeordnet ist.

2. Komponentenanordnung (1) nach Anspruch 1, **dadurch gekennzeichnet, dass** der erste RFID-Transponder (3) und/oder der zweite RFID-Transponder (6) eindeutige Komponentenidentifikationsdaten (25) bereitstellen.

3. Komponentenanordnung (1) nach Anspruch 1 oder 2, **dadurch gekennzeichnet, dass** der erste RFID-Transponder (3) biokompatibel ist.

4. Komponentenanordnung (1) nach einem der vorangegangenen Ansprüche, **dadurch gekennzeichnet, dass** der erste RFID-Transponder (3) mittels eines Sterilisationsverfahrens sterilisierbar ist.

5. Komponentenanordnung (1) nach einem der vorangegangenen Ansprüche, **dadurch gekennzeichnet, dass** die Komponente (2) ein Applikator (20) für die Röntgentherapie ist.

6. Röntgentherapiesystem (10), umfassend:
eine Röntgentherapieeinrichtung (11) mit einer Halteeinrichtung (12), eingerichtet zum Halten und/oder Positionieren mindestens einer Komponente (2) einer Komponentenanordnung (1) nach einem der Ansprüche 1 bis 5 in und/oder an einem Patienten,
eine Bedieneinrichtung (13), eingerichtet zum Steuern der
Röntgentherapieeinrichtung (11),
wobei die Röntgentherapieeinrichtung (11) mindestens einen ersten RFID-Leser (14) umfasst, der dazu eingerichtet ist, Daten eines an oder in der mindestens einen Komponente (2) der Komponentenanordnung (1) angeordneten ersten RFID-Transponders (3) auszulesen und/oder diesen mit Daten zu beschreiben, und
wobei die Bedieneinrichtung (13) mindestens einen zweiten RFID-Leser (16) umfasst, der dazu eingerichtet ist, Daten eines in und/oder an einer Sekundärverpackung (5) der Komponentenanordnung (1) nach einem der Ansprüche 1 bis 5 angeordneten zweiten RFID-Transponders (6) auszulesen und/oder diesen mit Daten zu beschreiben,
wobei das Röntgentherapiesystem (10) dazu eingerichtet ist, gemäß einem Verfahren nach einem der Ansprüche 8 bis 12 eingerichtet zu werden.

7. Röntgentherapiesystem (10) nach Anspruch 6, **dadurch gekennzeichnet, dass** der mindestens eine erste RFID-Leser (3) an der Halteeinrichtung (12) in einem Verbindungsbereich (17) angeordnet ist, in dem die mindestens eine Komponente (2) zur Nutzung an der Halteeinrichtung (12) angeordnet ist oder angeordnet werden kann.

8. Verfahren zum Betreiben eines Röntgentherapiesystems (10),
wobei zum Einrichten des Röntgentherapiesystems (10) für jede von mindestens einer in einer Komponentenanordnung (1) gemäß einem der Ansprüche 1 bis 5 vorliegenden Komponente (2):
die in einer Primärverpackung (4) steril verpackte Komponente (2) aus der Primärverpackung (4) entnommen wird, und
Daten eines ersten RFID-Transponders (3) der Komponente (2) mittels eines ersten RFID-Lesers (14) einer Röntgentherapieeinrichtung (11) des Röntgentherapiesystems (10) ausgelesen werden und/oder der erste RFID-Transponder (3) mit Daten beschrieben wird,
**dadurch gekennzeichnet, dass**
Daten eines in und/oder an einer Sekundärverpackung (5) der Komponente (2) angeordneten zweiten RFID-Transponders (6) mittels eines zweiten RFID-Lesers (16) einer Bedieneinrichtung (13) des Röntgentherapiesystems (10) ausgelesen werden und/oder der zweite RFID-Transponder (6) mit Daten beschrieben wird.

9. Verfahren nach Anspruch 8, **dadurch gekennzeichnet, dass** eine Freigabe der Röntgentherapie erfolgt, wenn die Daten des ersten RFID-Transponders (3) der mindestens einen Komponente (2) mindestens eine vorgegebene Information umfassen.

10. Verfahren nach Anspruch 8 oder 9, **dadurch gekennzeichnet, dass** nach einem Benutzen der mindestens einen Komponente (2) eine Information in dem ersten RFID-Transponder (3) hinterlegt wird, die die mindestens eine Komponente (2) als benutzt markiert und/oder die eine Anzahl von Benutzungen der mindestens einen Komponente (2) umfasst.

11. Verfahren nach einem Ansprüche 8 bis 10, **dadurch gekennzeichnet, dass** der erste RFID-Transponder (3) nach dem Benutzen und/oder nach Erreichen einer vorgegebenen Anzahl von Benutzungen unbrauchbar gemacht wird.

12. Verfahren nach einem der Ansprüche 8 bis 11, **dadurch gekennzeichnet, dass** die mindestens eine Komponente (2) nach einer Benutzung sterilisiert wird.

## Claims

1. Component arrangement (1) for an x-ray therapy component (2), comprising:
an x-ray therapy component (2) which has a first RFID transponder (3),
a primary packaging (4) in which the component (2) is packaged sterilely,
**characterized by**
a secondary packaging (5) which has a second RFID transponder (6) arranged therein and/or thereon and in which the component (2) packaged in the primary packaging (4) is arranged.

2. Component arrangement (1) according to Claim 1, **characterized in that** the first RFID transponder (3) and/or the second RFID transponder (6) provide unique component identification data (25).

3. Component arrangement (1) according to Claim 1 or 2, **characterized in that** the first RFID transponder (3) is biocompatible.

4. Component arrangement (1) according to any of the preceding claims, **characterized in that** the first RFID transponder (3) is sterilizable by means of a sterilization method.

5. Component arrangement (1) according to any of the preceding claims, **characterized in that** the component (2) is an applicator (20) for x-ray therapy.

6. X-ray therapy system (10), comprising:
an x-ray therapy device (11) having a holding device (12) configured to hold and/or position at least one component (2) of a component arrangement (1) according to any of Claims 1 to 5 in and/or on a patient,
an operating device (13) configured to control the x-ray therapy device (11),
wherein the x-ray therapy device (11) comprises at least one first RFID reader (14) configured to read-out data from a first RFID transponder (3) arranged on or in the at least one component (2) of the component arrangement (1) and/or write data to said first RFID transponder, and wherein the operating device (13) comprises at least one second RFID reader (16) configured to read-out data from a second RFID transponder (6) arranged in and/or on a secondary packaging (5) of the component arrangement (1) according to any of Claims 1 to 5 and/or write data to said second RFID transponder,
wherein the x-ray therapy system (10) is configured to be configured according to a method according to any of Claims 8 to 12.

7. X-ray therapy system (10) according to Claim 6, **characterized in that** the at least one first RFID reader (3) is arranged on the holding device (12) in a connection region (17) in which, for use purposes, the at least one component (2) is arranged or can be arranged on the holding device (12).

8. Method for operating an x-ray therapy system (10), wherein configuring the x-ray therapy system (10) for each of at least one component (2) present in a component arrangement (1) according to any of Claims 1 to 5 includes:
the component (2) sterilely packaged in a primary packaging (4) being taken from the primary packaging (4), and
a first RFID reader (14) of an x-ray therapy device (11) of the x-ray therapy system (10) being used to read-out data from a first RFID transponder (3) of the component (2) and/or data being written to the first RFID transponder (3),
**characterized in that**
a second RFID reader (16) of an operating device (13) of the x-ray therapy system (10) being used to read-out data from a second RFID transponder (6) arranged in and/or on a secondary packaging (5) of the component (2) and/or data being written to the second RFID transponder (6).

9. Method according to Claim 8, **characterized in that** clearance is given to the x-ray therapy if the data from the first RFID transponder (3) of the at least one component (2) comprise at least one piece of specified information.

10. Method according to Claim 8 or 9, **characterized in that** a use of the at least one component (2) is followed by storing a piece of information in the first RFID transponder (3), the information marking the at least one component (2) as used and/or comprising a number of uses of the at least one component (2).

11. Method according to any of Claims 8 to 10, **characterized in that** the first RFID transponder (3) is rendered unusable after use and/or once a given number of uses have been reached.

12. Method according to any of Claims 8 to 11, **characterized in that** the at least one component (2) is sterilized after one use.

## Revendications

1. Agencement de composants (1) pour un composant (2) de radiothérapie, comprenant :
un composant (2) pour la radiothérapie avec un premier transpondeur RFID (3),
un emballage primaire (4) dans lequel le composant (2) est emballé de manière stérile,
**caractérisé par**
un emballage secondaire (5) avec un deuxième transpondeur RFID (6) disposé dans et/ou sur celui-ci, dans lequel est disposé le composant (2) emballé dans l'emballage primaire (4).

2. Agencement de composants (1) selon la revendication 1, **caractérisé en ce que** le premier transpondeur RFID (3) et/ou le deuxième transpondeur RFID (6) fournissent des données univoques d'identification (25) de composants.

3. Agencement de composants (1) selon la revendication 1 ou 2, **caractérisé en ce que** le premier transpondeur RFID (3) est biocompatible.

4. Agencement de composants (1) selon l'une quelconque des revendications précédentes, **caractérisé en ce que** le premier transpondeur RFID (3) est stérilisable au moyen d'un procédé de stérilisation.

5. Agencement de composants (1) selon l'une quelconque des revendications précédentes, **caractérisé en ce que** le composant (2) est un applicateur (20) pour la radiothérapie.

6. Système de radiothérapie (10), comprenant :
un dispositif de radiothérapie (11) comportant un dispositif de maintien (12), conçu pour le maintien et/ou le positionnement d'au moins un composant (2) d'un agencement de composants (1) selon l'une quelconque des revendications 1 à 5 dans et/ou sur un patient,
un dispositif de commande (13), conçu pour la commande du dispositif de radiothérapie (11),
le dispositif de radiothérapie (11) comprenant au moins un premier lecteur RFID (14) qui est conçu pour lire des données d'un premier transpondeur RFID (3) disposé sur ou dans ledit au moins un composant (2) de l'agencement de composants (1) et/ou pour inscrire des données sur ce transpondeur, et
le dispositif de commande (13) comprenant au moins un deuxième lecteur RFID (16) qui est conçu pour lire des données d'un deuxième transpondeur RFID (6) disposé dans et/ou sur un emballage secondaire (5) de l'agencement de composants (1) selon l'une quelconque des revendications 1 à 5 et/ou pour inscrire des données sur ce transpondeur, le système de radiothérapie (10) étant conçu pour être agencé conformément à un procédé selon l'une quelconque des revendications 8 à 12.

7. Système de radiothérapie (10) selon la revendication 6, **caractérisé en ce que** ledit au moins un premier lecteur RFID (3) est disposé sur le dispositif de maintien (12) dans une zone de connexion (17) dans laquelle ledit au moins un composant (2) est disposé ou peut être disposé pour l'utilisation sur le dispositif de maintien (12).

8. Procédé pour faire fonctionner un système de radiothérapie (10),
dans lequel, pour l'agencement du système de radiothérapie (10) pour chacun d'au moins un composant (2) présent dans un agencement de composants (1) selon l'une quelconque des revendications 1 à 5 :
le composant (2) emballé de manière stérile dans un emballage primaire (4) est retiré de l'emballage primaire (4), et
des données d'un premier transpondeur RFID (3) du composant (2) sont lues au moyen d'un premier lecteur RFID (14) d'un dispositif de radiothérapie (11) du système de radiothérapie (10) et/ou des données sont inscrites sur le premier transpondeur RFID (3),
**caractérisé en ce que**
des données d'un deuxième transpondeur RFID (6) disposé dans et/ou sur un emballage secondaire (5) du composant (2) sont lues au moyen d'un deuxième lecteur RFID (16) d'un dispositif de commande (13) du système de radiothérapie (10) et/ou des données sont inscrites sur le deuxième transpondeur RFID (6).

9. Procédé selon la revendication 8, **caractérisé en ce qu'**une autorisation de la radiothérapie a lieu lorsque les données du premier transpondeur RFID (3) dudit au moins un composant (2) comprennent au moins une information préétablie.

10. Procédé selon la revendication 8 ou 9, **caractérisé en ce qu'**après une utilisation dudit au moins un composant (2), est déposée dans le premier transpondeur RFID (3) une information qui marque comme utilisé ledit au moins un composant (2) et/ou qui comprend un nombre d'utilisations dudit au moins un composant (2).

11. Procédé selon l'une quelconque des revendications 8 à 10, **caractérisé en ce que** le premier transpondeur RFID (3) est rendu inutilisable après avoir été utilisé et/ou après avoir atteint un nombre préétabli d'utilisations.

12. Procédé selon l'une quelconque des revendications 8 à 11, **caractérisé en ce que** ledit au moins un composant (2) est stérilisé après une utilisation.
